(19) **Europäisches Patentamt / European Patent Office / Office européen des brevets**

(11) **EP 3 982 925 B1**

(12) **FASCICULE DE BREVET EUROPEEN**

(45) Date de publication et mention de la délivrance du brevet:
**07.06.2023 Bulletin 2023/23**

(21) Numéro de dépôt: **20742329.4**

(22) Date de dépôt: **12.06.2020**

(51) Classification Internationale des Brevets (IPC):
*A61K 41/00* (2020.01)   *A61K 9/00* (2006.01)
*A61K 9/51* (2006.01)   *A61K 47/60* (2017.01)
*C12Q 1/6816* (2018.01)   *G01N 33/543* (2006.01)
*G01N 33/58* (2006.01)

(52) Classification Coopérative des Brevets (CPC):
(C-Sets disponibles)
**A61K 41/0052; A61K 41/0038; C12Q 1/6816; G01N 33/54346; G01N 33/587;** G01N 2610/00

(Cont.)

(86) Numéro de dépôt international:
**PCT/FR2020/051005**

(87) Numéro de publication internationale:
**WO 2020/249912 (17.12.2020 Gazette 2020/51)**

(54) **NANOPARTICULES PREFONCTIONNALISEES A L'AIDE D'UNE MONOCOUCHE AUTO-ASSEMBLEE ET LEUR METHODE DE PREPARATION**

PRE-FUNKTIONALISIERTE NANOPARTIKEL MIT EINER SELBSTORGANISIERTEN MONOLAGE UND IHRE HERSTELLUNGSVERFAHREN

PREFUNCTIONALISED NANOPARTICLES WITH A SELF-ASSEMBLED MONOLAYER AND THEIR METHOD OF PREPARATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.06.2019 FR 1906224**

(43) Date de publication de la demande:
**20.04.2022 Bulletin 2022/16**

(73) Titulaires:
• **Centre national de la recherche scientifique**
**75016 Paris (FR)**
• **École Normale Supérieure Paris-Saclay**
**94235 Cachan (FR)**

(72) Inventeurs:
• **NOGUES, Claude**
**92160 ANTONY (FR)**
• **BUCKLE, Malcolm**
**92160 ANTONY (FR)**
• **VIAL, Stéphanie**
**PARIS 75015 (FR)**

(74) Mandataire: **IP Trust**
**2, rue de Clichy**
**75009 Paris (FR)**

(56) Documents cités:
CN-A- 106 581 696      FR-A1- 2 863 053
US-A1- 2011 165 077      US-A1- 2016 243 254
US-A1- 2016 274 086      US-A1- 2019 142 966

• WANG WEI ET AL: "Role of thiol-containing polyethylene glycol (thiol-PEG) in the modification process of gold nanoparticles (AuNPs): Stabilizer or coagulant?", JOURNAL OF COLLOID AND INTERFACE SCIENCE, ACADEMIC PRESS, INC, US, vol. 404, 30 avril 2013 (2013-04-30), pages 223-229, XP028566628, ISSN: 0021-9797, DOI: 10.1016/J.JCIS.2013.04.020

EP 3 982 925 B1

**(Cont. page suivante)**

- **KENDALL M HURST ET AL: "Self-Assembled Monolayer-Immobilized Gold Nanoparticles as Durable, Anti-Stiction Coatings for MEMS", JOURNAL OF MICROELECTROMECHANICAL SYSTEMS, IEEE SERVICE CENTER, US, vol. 20, no. 2, 1 avril 2011 (2011-04-01), pages 424-435, XP011352660, ISSN: 1057-7157, DOI: 10.1109/JMEMS.2011.2112334**

(52) Classification Coopérative des Brevets (CPC):
(Cont.)

C-Sets
**C12Q 1/6816, C12Q 2563/155, C12Q 2565/519, C12Q 2565/628**

## Description

La présente invention a trait au domaine des nanoparticules (NP) préfonctionnalisées. Elle concerne plus particulièrement des NP préfonctionnalisées à l'aide d'une monocouche auto-assemblée (SAM) ainsi que des NP fonctionnalisées à l'aide de biomolécules de sorte à ce que lesdites NP soient stables en solution. Ces NP peuvent être utilisées dans de nombreuses applications, notamment en tant qu'outil de diagnostic, outil de déplétion ou de concentration d'une molécule d'intérêt dans une solution, et outil thérapeutique.

## Etat de la technique et inconvénients

Des nanoparticules recouvertes d'une couche SAM sont décrites dans la littérature. Par exemple, l'article de Hurst K.M. et al. (Journal of Microelectromechanical Systems ; Volume: 20, Issue: 2 , April 2011) décrit l'immobilisation d'une SAM constituée de p-aminophenyl trimethoxysilane (APhTS) et de 3-mercaptopropyl triméthoxysilane (MPTS) sur des NP afin de stabiliser l'adhésion de ces NP sur des surfaces siliconées de systèmes microélectromécaniques. Toutefois, de telles NP ne sont pas adaptées à une fonctionnalisation avec des biomolécules.

D'autres documents de l'art antérieur décrivent des nanoparticules recouvertes d'une monocouche thiolée.

Hurst et al. (2006) décrit des nanoparticules d'or sur lesquelles sont chargées des molécules d'ADN, l'objectif de cette étude étant de charger un maximum de molécules d'ADN à la surface des nanoparticules. Les auteurs montrent que la charge maximale est obtenue lorsque les conditions de salinité sont maîtrisées (0,7 M NaCl) et que les molécules d'ADN contiennent un espaceur de type polyéthylène glycol (PEG).

Wang et al. (RSC Adv. 2017, 7. 3676-3679) décrit des NP d'or sphériques fonctionnalisées à l'aide d'une monocouche mixte comprenant des PEG et de l'ADN thiolé. Ces nanoparticules sont préparées en 2 étapes : fonctionnalisation avec les molécules d'ADN thiolé puis avec les PEG thiolés de sorte à recouvrir la totalité de la surface de la NP.

Li et al. (Langmuir 2015, DOI: 10.1021/acs.langmuir.5b01680) décrit des nanobâtonnets (NR) fonctionnalisées à l'aide de molécules d'ADN. Les NR d'or étant chargés positivement, elles ont tendance à s'agréger en présence d'ADN chargé négativement. Les auteurs proposent de greffer sur les NR de longues molécules de PEG et un surfactant préalablement à l'ajout d'ADN thiolé.

Le document US 2011/165077 décrit l'utilisation de nanoparticules d'or pour l'imagerie. Des molécules reportrices sont greffées sur la surface des NP, puis ces dernières sont recouvertes à saturation d'une couche protectrice PEG-SH ; cette couche évite l'agrégation des particules et peut être fonctionnalisée à l'aide de sondes spécifiques.

Le document US 2019/142966 décrit l'utilisation de NP métalliques destinées au traitement du cancer. Ces NP sont recouvertes de molécules PEG-COOH en une seule étape puis les groupes carboxy sont fonctionnalisés en présence de SDS avec des molécules thérapeutiques et des molécules de ciblage.

Le document US 2016/243254 décrit la préparation de nanosondes comme outils théranostiques dans le traitement du cancer. Une première couche de PEG-COOH thiolés est greffée à la surface de NP ; cette première couche recouvre environ 30% de la surface des NP, ce qui constitue un taux maximal de recouvrement du fait du poids moléculaire élevé des PEG-COOH. Une deuxième couche de molécules DNA-harpin thiolées est ajoutée et s'intercale entre les molécules de PEG. Dans cette configuration, la présence combinée d'un taux de recouvrement partiel et de la fonction carboxy est favorable à l'apparition d'interactions non spécifiques.

Dans le domaine des NP destinées à être utilisées en tant que support pour fixer des sondes biologiques, différentes contraintes doivent être considérées. Tout d'abord, les NP préfonctionnalisées ou fonctionnalisées doivent être stables en solution, c'est-à-dire qu'elles ne doivent pas s'agréger. Elles doivent également permettre une interaction sondes - molécules d'intérêt qui soit spécifique.

## Exposé de l'invention et avantages

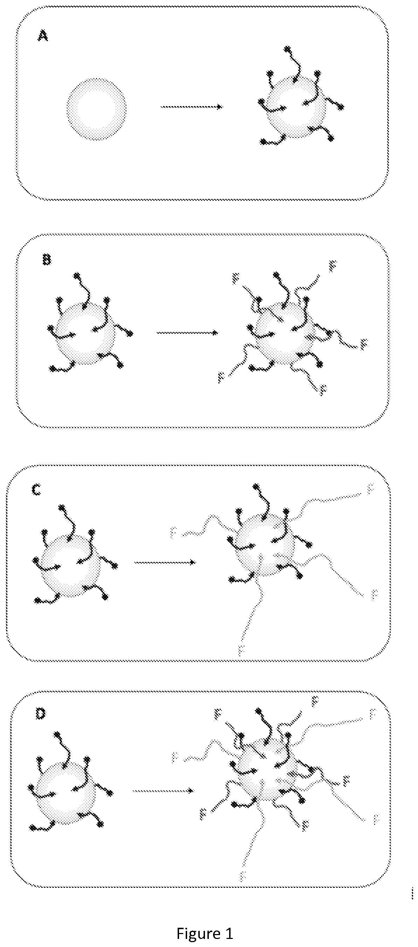

La présente invention apporte une solution à ces problèmes par la pré-fonctionnalisation des NP à l'aide d'une monocouche auto-assemblée constituée de molécules de poly-éthylène glycol (PEG) de faible poids moléculaire et dont le recouvrement est partiel. La présence de cette précouche à la surface des NP permet, seule ou en combinaison avec d'autres molécules (fonctionnalisation), de préparer des NP stables en solution. Cette monocouche a la particularité d'être constituée de molécules de faible poids moléculaire (compris entre 100 et 732 Dalton) dont les chaînes sont courtes ; l'encombrement au niveau de la surface est optimal, ce qui confère aux nanoparticules leurs propriétés innovantes décrites ci-après. Une représentation de la configuration des éléments greffés à la surface des NP est illustrée à la Figure 1.

La stratégie de fonctionnalisation selon l'invention est une chimie de surface qui permet d'améliorer la stabilité des nanoparticules métalliques, par exemple en or ou de nanoparticules hydride type cœur-coquille, dans des milieux tampons et complexes, en contrôlant le nombre de ligands (biomolécules) immobilisés à la surface des nanoparticules, en inhibant les interactions non-spécifiques et en optimisant les interactions spécifiques. Cette technologie consiste en une pré-fonctionnalisation avant l'immobilisation d'une ou plusieurs biomolécules servant de sondes. Soit les biomolécules possèdent un groupement thiol qui permet leur immobilisation directement sur la surface de la NP, soit l'immobilisation des biomolécules s'effectue en deux étapes : 1 - immobilisation d'une molécule de liaison possédant d'une part un thiol (pour son immobilisation sur la surface métallique) et d'autre part un groupement fonctionnel réactif (pour l'immobilisation des biomolécules), puis 2 - ajout d'une biomolécule (sonde ou molécule active) comportant un groupement capable de réagir ou interagir avec le groupement fonctionnel présent sur la molécule de liaison. La méthode décrite ici convient notamment à des NP sphériques, des nanobâtonnets, de nanocubes, des nanotriangles et des nanooursins.

De plus, cette pré-fonctionnalisation permet une optimisation de la densité des sondes, une répartition homogène des sondes et une absence d'interactions non spécifiques. En particulier, l'invention permet de disposer de NP fonctionnalisées à l'aide de sondes dont la densité est faible et optimisée pour obtenir une grande sensibilité de détection. Il est également possible de combiner plusieurs sondes différentes en conservant une densité et répartition contrôlées pour chacune des sondes.

## DESCRIPTION DETAILLEE DE L'INVENTION

Un premier objet de l'invention concerne une nanoparticule présentant une surface métallique pré-fonctionnalisée à l'aide d'une monocouche auto-assemblée (SAM) formée par une matrice de molécules portant une fonction thiol à l'une des extrémités, l'autre extrémité étant inerte, ladite nanoparticule étant caractérisée en ce que le taux de recouvrement W de ladite monocouche est compris entre 1,5% et 99%.

La particularité de la monocouche auto-assemblée est qu'elle est appliquée sur la surface des NP avant leur fonctionnalisation et qu'elle est insaturée. Elle joue un rôle de protection de la surface. Cette monocouche est constituée de molécules M de formule (I) :

$$HS(CH_2)_n (OCH_2CH_2)_m OH$$

dans laquelle :

n représente le nombre de $CH_2$ avec $3 \leq n \leq 11$
m représente le nombre d'éthylène glycol avec $1 \leq m \leq 12$.

Le poids moléculaire de la molécule M est compris entre 100 et 732 Dalton.

Globalement, un taux de recouvrement W de 1,5% minimum est nécessaire pour observer les effets de la couche SAM ; un faible taux de recouvrement permet d'avoir une forte densité de biomolécules à la surface de la NP, ce qui peut être intéressant par exemple lorsque 2 biomolécules différentes sont présentes. A l'opposé, le taux de recouvrement W peut être élevé, proche de la saturation (99% par exemple) - permettant le dépôt d'une molécule unique ; la seule limite de cette approche peut être celle de la détection, par manque d'outil assez sensible. Dans tous les cas, le fait de pré-fonctionnaliser la surface de la NP permet d'immobiliser les biomolécules de manière contrôlée, c'est-à-dire d'assurer que la distance moyenne entre les biomolécules immobilisées est homogène.

Dans des modes de réalisation particuliers, le taux de recouvrement W peut être compris entre 3% et 80%, par exemple entre 10% et 50%, ou entre 5% et 30% en fonction des applications, notamment de 2%, 5%, 10%, 20%, 30%, 40%, 50%,60%, 70%, 80%, 90%, 95%, 98%.

Par « taux de recouvrement » au sens de l'invention, on entend un taux de recouvrement théorique initial ; celui-ci correspond à la surface de la molécule et se calcule selon la formule (2) suivante :

$$(empreinte\ exprimée\ en\ nm^2) \times 100\ /\ surface\ de\ la\ nanoparticule$$

Au sens de l'invention, une « nanoparticule » (NP) peut être une nanoparticule sphérique (NS), un nanobâtonnet (NR), une nanoparticule cubique (NC) ou un nanotriangle (NT) ou un nano-oursin. Il peut s'agir en particulier de nanoparticules d'or sphériques dont le diamètre est compris entre 10 et 80 nm, de nanobâtonnets dont le rapport de forme est compris entre 2,5 et 5, de nanocubes de 40 nm d'arête, ou de nanotriangles dont les côtés sont compris entre 30 et 100 nm et d'épaisseur 10 - 40 nm. Les NP peuvent aussi être des NP hybrides de même forme (sphères, nanobâtonnets...) possédant par exemple un coeur de type oxyde (ex : oxyde de fer, oxyde de silicium) ou métallique et une coquille métallique (ex : or). La surface métallique des nanoparticules hybrides coeur-coquille est similaire à la surface des nanoparticules métalliques, et le mode de calcul de leur surface est identique.

La surface de la molécule dépend de sa forme et celle-ci doit être connue pour déterminer le taux de recouvrement.

Dans le cas d'une nanoparticule sphérique, la surface correspond à la formule (3) suivante :

$4\pi r^2$

avec r correspondant au rayon de la nanoparticule.

Dans le cas d'un nanôbatonnet, la surface correspond à la formule (4) suivante :

$2\pi rl + 2\pi r^2$

avec r correspondant au rayon du nanôbatonnet et l correspondant à sa longueur.

Dans le cas d'un nanocube, la surface correspond à la formule (5) suivante :

$6a^2$

avec a correspondant à l'arête du nanocube.

Dans le cas d'un nanotriangle, la surface correspond à la formule (6) suivante :

$(ph/2) + b$

avec p correspondant au périmètre de la base, h à la hauteur de la pente et b à l'aire de la base.

Les molécules présentes à la surface des NP pour leur pré-fonctionnalisation ou leur fonctionnalisation peuvent être choisies parmi les molécules suivantes : molécules M telles que définies précédemment, molécules L-PEG et PEG-F telles que définies ci-après et les ADN double brin (dsDNA) ou simple brin (ssDNA), ARN, protéines telles que les anticorps ou peptides, aptamères...

Ainsi, pour déterminer le taux de recouvrement, il faut également connaître les empreintes théoriques des molécules présentes à la surface des NP :

$$\text{Empreinte de M et PEG-F} = 0{,}23 \text{ nm}^2 \quad [1]$$

$$\text{Empreinte L-PEG} = 0{,}45 \text{ nm}^2 \quad [2]$$

Empreinte dsADN = 3,14 nm$^2$

Dans un mode de réalisation avantageux, les nanoparticules pré-fonctionnalisées selon l'invention sont fonctionnalisées à l'aide d'au moins une molécule L-PEG dont le taux de recouvrement X est d'au moins 1%, de préférence compris entre 1% et 10%.

Par « molécule L-PEG », on entend une molécule de polyéthylène glycol thiolée de longue taille et de haut poids moléculaire.

Les molécules L-PEG peuvent être de formule (7):

$$\text{SH-CH}_2\text{CH}_2(\text{CH}_2\text{CH}_2\text{O})_q\text{O-F}$$

dans laquelle :

q représente le nombre d'éthylène glycol avec $20 \leq q \leq 500$;
F représente un groupement fonctionnel tel que $CH_3$, OH, COOH, $NH_2$, ....

Le poids moléculaire de la molécule L-PEG est supérieur ou égal 800 Dalton.

La fonctionnalisation à l'aide de molécules L-PEG est particulièrement intéressante pour stabiliser les NP dans une solution de PBS. Elle peut être combinée avec une pré-fonctionnalisation à l'aide de molécules M pour préparer des NP « prêtes à l'emploi » sur lesquelles une ou plusieurs biomolécules peuvent être adsorbées.

De plus, lorsque le groupement F est autre que $CH_3$ ou OH, celui-ci peut interagir et/ou réagir avec des biomolécules afin d'ajouter des sondes en surface des NP.

Dans un mode de réalisation particulier de l'invention, les nanoparticules pré-fonctionnalisées selon l'invention sont fonctionnalisées à l'aide d'au moins une molécule thiolée, ladite molécule thiolée étant :

- soit une molécule de liaison thiolée comportant une fonction thiol à l'une des extrémités et portant à son autre extrémité un groupement actif ou réactif capable d'interagir avec une biomolécule ;
- soit une biomolécule comportant une fonction thiol.

Ces nanoparticules peuvent en outre être fonctionnalisées à l'aide de molécule L-PEG tel que décrit précédemment.

Dans un mode de réalisation d'intérêt, la molécule de liaison thiolée est une molécule PEG-F dans lequel F est un groupement fonctionnel activable, tel un COOH et dont le taux de recouvrement Y est au minimum de 1%. Le taux de recouvrement global de la surface de la NP peut être partiel ou total (saturation) en fonction des applications.

Dans un mode de réalisation préféré, le recouvrement global est partiel.

Par « PEG-F », on entend une molécule de polyéthylène glycol thiolée.

Les molécules PEG-F peuvent par exemple être de formule (8):

$$HS(CH_2)_r \, (OCH_2CH_2)_p \, O\text{-}F$$

dans laquelle :

r représente le nombre de $CH_2$ et est un nombre entier supérieur ou égal à 3 ;
p représente le nombre d'éthylène glycol avec $2 \leq p \leq 12$, et
F représente un groupement fonctionnel tel que $CH_3$, COOH, $NH_2$, ....

Le poids moléculaire de la molécule PEG-F peut être bas ou haut allant jusqu'à plus de 800 kDa.

Dans un mode de réalisation alternatif, les molécules de liaison peuvent être des molécules L-PEG comportant un groupement fonctionnel activable.

Dans un autre mode de réalisation d'intérêt, les nanoparticules pré-fonctionnalisées selon l'invention sont fonctionnalisées à l'aide d'une biomolécule thiolée servant de sonde (appelées « sondes thiolées ») telle des acides nucléiques (ADN, ARN, PNA....), des protéines (anticorps, antigènes, hormones,...), des peptides, des hormones, des sucres, des acides gras ou encore des cellules entières.

Dans un autre mode de réalisation d'intérêt, les biomolécules thiolées sont des petites molécules comme des drogues, intercalant, ou complexant, appelées ici « molécules actives ». Ces molécules actives peuvent également être liées par l'intermédiaire d'un groupement fonctionnel activable présent sur les molécules de PEG-F ou de L-PEG.

Lorsque la molécule thiolée est un ADN double brin ou simple, le taux de recouvrement Z est minimum 10%. Le taux de recouvrement global de la surface de la NP peut être partiel ou total (saturation) en fonction des applications. Dans un mode de réalisation préféré, le recouvrement global est partiel.

Dans un mode de réalisation préféré, lorsque le PEG-F est court, des molécules L-PEG sont ajoutées à raison de 1% pour stabiliser les NP.

Par « sonde thiolée » au sens de l'invention, on entend une biomolécule jouant le rôle de sonde apte à interagir spécifiquement avec (à capter) une molécule d'intérêt se trouvant dans un milieu aqueux (solution tampon par exemple) ou complexe (milieu de culture, milieu biologique, fluides corporels, matrice liquide telle que le sang, le plasma, le sérum, l'urine, les larmes, les extraits cellulaires...). Ces biomolécules servant de sondes sont fixées au support de la NP (i) soit par interaction directe d'un groupement thiolé présent sur les biomolécules avec la surface de la NP, (ii) soit par l'intermédiaire d'une molécule thiolée à l'une de ses extrémités et portant à l'autre extrémité un groupement actif ou réactif capable de réagir avec (de se lier avec) la biomolécule par la formation d'une liaison « forte » (par exemple une liaison covalente comme la liaison amide, une chélation, une affinité forte de type « tag » comme la liaison streptavidine-biotine); cette molécule thiolée est ici appelée « molécule de liaison ».

Une fois que le substrat est protégé par la couche protectrice SAM comme décrit dans ce qui précède, on procède, immédiatement ou extemporanément après quelques heures, jours ou mois, à une étape additionnelle de dépôt de sondes thiolées.

Ces sondes thiolées peuvent être :

- des sondes directement thiolées, nécessitant un temps d'incubation qui dépend de la concentration, qui est généralement faible (de l'ordre de la micromolaire ou à concentration plus faible) ;
- des molécules thiolées, présentant un groupement réactif amine, carboxyle, double ou triple liaison carbone-carbone, epoxy, chimie click...
- des molécules thiolées, présentant un groupement actif interagissant avec des molécules complémentaires pour former un sandwich, par exemple biotine- streptavidine-biotine...

Un deuxième objet de l'invention concerne un procédé de préparation d'une nanoparticule présentant une surface métallique pré-fonctionnalisée à l'aide d'une monocouche auto-assemblée (SAM) formée par une solution de molécules portant une fonction thiol à l'une des extrémités, l'autre extrémité étant inerte, comprenant les étapes suivantes :

- Ajouter des molécules M aptes à former une monocouche auto-assemblée, de sorte à obtenir un taux de recouvrement W compris entre 1,5 % et 99%,
- Agiter pendant une durée d'au moins 5 minutes.

Un tel procédé permet de préparer des NP pré-fonctionnalisées, stables dans l'eau et dans le PBS. Les NP ainsi préparées peuvent être conservées pendant plusieurs mois à 4°C jusqu'à utilisation.

Il est possible d'ajouter un surfactant au cours de ce procédé. Le surfactant utilisé peut être choisi parmi tous les surfactants connus de l'homme du métier, en particulier le SDS, CHAPS, NP40, Tween 20 (SDS: sodium dodecyl sulfate, CHAPS :3-[(3-Cholamidopropyl)dimethylammonio]-1-propanesulfonate)... L'homme du métier est familier de l'utilisation de surfactants pour la préparation de NP en solution.

Dans un mode de réalisation particulier de l'invention, on ajoute en outre à la solution de NP pré-fonctionnalisées à l'aide de la couche SAM, une solution de molécules L-PEG pour obtenir un taux de recouvrement Y compris entre 1 et 10%, on incube sous agitation pendant une durée minimum de 5 min, généralement comprise entre 5 min et 1h.

Les NP pré-fonctionnalisées à l'aide de molécules M et éventuellement fonctionnalisées à l'aide de molécules L-PEG, peuvent en outre être fonctionnalisées à l'aide de molécules thiolées et/ou de biomolécules. Dans un mode de réalisation alternatif, les molécules L-PEG peuvent être ajoutées après les molécules PEG-F.

Un tel procédé de préparation comprend les étapes suivantes :

- Ajouter une molécule thiolée choisie parmi une molécule de liaison thiolée apte à interagir avec une biomolécule ou une biomolécule thiolée,
- Agiter par exemple pendant une durée comprise entre 5 min et 24h,
- Si la molécule thiolée est une biomolécule, se mettre dans les conditions tampons adaptées à cette dernière,
- Enlever l'excès de molécule thiolée, par exemple par centrifugation,
- Redisperser dans une solution appropriée (eau distillée, solution tampon avec surfactant...),
- Si la molécule thiolée est une molécule activable, ajouter l'activateur (EDC/NHS ou PDEA par exemple), incuber pendant au moins 5 min, centrifuger et redisperser dans une solution tampon avant d'incuber avec une concentration connue de biomolécules à immobiliser,
- Si la molécule thiolée est une molécule de liaison thiolée, ajouter éventuellement ensuite une solution de biomolécules aptes à interagir avec le groupement de la molécule de liaison.

Ce procédé permet de préparer différents types de NP fonctionnalisées en fonction des étapes réalisées :

- NP pré-fonctionnalisées M
- NP pré-fonctionnalisées M + L-PEG
- NP pré-fonctionnalisées M + PEG-F
- NP pré-fonctionnalisées M + L-PEG + PEG-F
- NP pré-fonctionnalisées M + biomolécule thiolée (ex: ADN)
- NP pré-fonctionnalisées M + L-PEG + biomolécule thiolée

et ainsi de suite avec tout type de biomolécules appropriées à un usage en tant que sonde.

Des exemples de modes de réalisation sont présentés à la Figure 1.

L'avantage du procédé qui débute avec la préparation de NP pré-fonctionnalisées avec la molécule M à différents taux de recouvrement est que les NPs sont stables pour les étapes suivantes de fonctionnalisation, qui peuvent se faire dans l'eau et aussi dans des solutions tampons. Ces molécules M sont des petites molécules courtes, de faible poids moléculaire et inertes. La pré-fonctionnalisation avec la molécule M assure que la fonctionnalisation est homogène sur l'ensemble de la surface des NP quelle que soit leur taille et leur forme. De plus, elle assure que les biomolécules immobilisées ne sont pas dénaturées et sont orientées vers la solution et non pas vers la surface de la NP. D'autre part, la molécule M empêche les interactions non spécifiques.

Un troisième objet de l'invention concerne l'utilisation des nanoparticules selon l'invention.

Les NP fonctionnalisées peuvent être utilisées dans de nombreuses applications bien connues de l'homme du métier dans le domaine médical, agroalimentaire et environnemental.

Elles peuvent notamment être utilisées pour détecter des molécules d'intérêt en solution, typiquement dans des tests de diagnostic.

Ainsi l'invention concerne une méthode de détection d'une molécule d'intérêt dans une solution, en particulier un milieu complexe, comprenant les étapes de :

- mise en contact d'une solution avec au moins une nanoparticule fonctionnalisée telle que définie précédemment.
- détection par SPR, test bandelette ou toute autre méthode appropriée, d'un signal spécifique lorsqu'une biomolécule interagit avec l'un des composants de la solution.

Les NP fonctionnalisées selon l'invention peuvent également être utilisées pour dépléter des molécules particulières, que ce soit des molécules d'intérêt que l'on souhaite récupérer ou bien des molécules indésirables que l'on souhaite éliminer.

Ainsi, l'invention concerne une méthode de déplétion de molécules d'intérêt présentes dans une solution, en particulier un milieu complexe, comprenant les étapes de :

a) mise en contact d'une solution contenant la molécule d'intérêt avec une nanoparticule fonctionnalisée telle que définie précédemment,
b) incubation de ladite solution en présence desdites nanoparticules,
c) récupération des nanoparticules,
d) optionnellement, répéter les étapes a) à c) jusqu'à épuisement de la solution en molécule d'intérêt.

L'utilisation des NP fonctionnalisées pour permettre leur ciblage aux cellules cibles et/ou le greffage de composés thérapeutiques est depuis longtemps envisagée dans le traitement des cancers. En radiothérapie, ces NP activables grâce aux rayons X pourraient constituer une avancée majeure dans la pratique de la radiothérapie visant à détruire des tumeurs sous contrôle. Les NP peuvent également être utilisées dans des approches de photothermie, une des applications les plus prometteuses des nanoparticules d'or pour lutter contre le cancer. L'idée est d'injecter des nanoparticules d'or dans la circulation sanguine des patients. Ces nanoparticules, par leur structure, ont une tendance à se fixer dans les tissus cancéreux, mais il est possible de les diriger encore plus spécifiquement vers la tumeur en les recouvrant de molécules particulières (fonctionnalisation). Elles sont ensuite « chauffées » à l'aide d'une lumière laser. La chaleur dégagée par les NP va créer des lésions irréversibles dans les cellules cancéreuses. Cette technique est actuellement testée dans plusieurs essais cliniques dans les cancers de la tête, du cou, ou encore du poumon et de la prostate.

Ainsi, l'invention concerne l'utilisation d'une NP fonctionnalisée selon l'invention dans le traitement du cancer, notamment par radiothérapie ou photothermie. La photothermie contrôlée pourrait permettre le relargage d'une molécule thérapeutique préalablement greffée sur la nanoparticule sans affecter la cellule ou l'organe cible.

L'invention concerne l'utilisation d'une NP fonctionnalisée selon l'invention pour l'imagerie médicale.

La solution dans laquelle sont ajoutées les NP peut être de nature très diverse. Il peut s'agir de milieux simples (eau, PBS, milieux modèles) ou de milieux complexes (fluides corporels, eaux usées, jus...). Il peut aussi s'agir plus généralement d'une matrice dans laquelle, ou au contact de laquelle, les NP peuvent être dispersées. Cette matrice peut être un gel, du sable, ou toute surface plane susceptible de présenter des molécules d'intérêt à sa surface.

Dans un mode de réalisation particulier, la solution est un milieu biologique complexe tel qu'un extrait cellulaire, un extrait de culture bactérienne, un échantillon biologique humain choisi parmi du sérum, du sang, de l'urine, du liquide amniotique, des larmes..., un milieu aquatique choisi parmi des eaux usées, des eaux polluées ou susceptibles de l'être, de l'eau de mer, l'eau d'un aquarium...

**BREVE DESCRIPTION DES FIGURES**

**Figure 1 :** Représentation de la technologie pour la fonctionalisation de NP. A) Préfonctionalisation avec une SAM composée de molécules M (représentées par une courbe courte noire) ; B) M + L-PEG (représenté par une longue courbe gris clair) ; C) M + PEG-F ou biomolécule (représenté(e) par une courbe de longueur intermédiaire gris moyen) ; D) M + PEG-F ou biomolécule + L-PEG. F représente un groupement fonctionnel.

**Figure 2** : Spectres d'absorbance de NS pré-fonctionnalisées avec des molécules M en fonction du taux de recouvrement, dans PBS en présence ou absence de surfactant. Nanoparticules sphériques de 20 nm ; temps agitation = 4h, M (n = 11, m = 4).

**Figure 3** : Spectres d'absorbance de NR pré-fonctionnalisées avec des molécules M en fonction du taux de recouvrement, dans PBS en présence de surfactant. Nanobâtonnets λ=800 nm ; temps agitation = 30 min, M (n = 11, m = 4), W = 8%.

**Figure 4** : Spectres d'absorbance de NS pré-fonctionnalisées avec des molécules M en fonction du taux de recouvrement dans PBS après centrifugation. Nanoparticules de 20 nm ; taux de recouvrement entre 1,6 et 80 %, temps agitation = 5 min, M (n = 11, m = 4), PBS + 0,005% Surfactant.

**Figure 5 :** Spectres d'absorbance de NS pré-fonctionnalisées avec des molécules M en fonction du taux de recouvrement dans PBS en présence de surfactant. Nanoparticules de 20 nm ; taux de recouvrement 1,6 et 80 %, temps agitation = 4h, M (n = 11, m = 4), PBS+0,005% surfactant.

**Figure 6 :** Spectres d'absorbance de NS pré-fonctionnalisées avec des molécules M en fonction du taux de recouvrement dans PBS en présence de surfactant. Nanoparticules de 40 nm commerciales (ref :741981 Sigma Aldrich) ; taux de recouvrement 0, 1,6 et 16 %, temps agitation = 4h, M (n = 11, m = 1.2), PBS+0,005% surfactant.

**Figure 7** : Spectre d'absorbance de NS pré-fonctionnalisées avec des molécules M et NP pré-fonctionnalisées avec des molécules M et fonctionnalisées avec les L-PEG(W = 5%) dispersées dans PBS avec différents taux de recouvrement de L-PEG (X = 0, 1, 10 %). Nanoparticule de 40 nm (ref : 741981 Sigma Aldrich), temps incubation L-PEG = 5 min, M (n = 11, m = 4), L-PEG poids moléculaire = 6000, F = $CH_3$.

**Figure 8** : Spectre d'absorbance de NS fonctionnalisées avec des molécules thiolées portant une fonction COOH (Y=1,6%) dispersées dans PBS avec différents taux de recouvrement de molécules M (W = 0, 1,6 et 16%). Nanoparticules de 20 nm. M (n = 11, m = 4) et PEG-COOH (r = 11, p=6).

**Figure 9** : Spectre d'absorbance de NS pré-fonctionnalisées avec des molécules M et fonctionnalisées PEG-COOH (W = 5%, Y = 5%) en fonction de la présence d'une fonctionnalisation L-PEG, dispersées dans du PBS avec différents taux de recouvrement de L-PEG (X = 0 ou 10 %) ; taille NS = 20 nm. M (n = 11, m = 4), L-PEG poids moléculaire = 6000 et F = CH3, PEG-COOH (r = 11, p - 6).

**Figure 10** : Spectre d'absorbance de NS pré-fonctionnalisées avec des molécules M et fonctionnalisées PEG-COOH (W = 5%, Y = 5%) dispersées dans du PBS avec différents taux de recouvrement de L-PEG (X = 0 ou 10 %) ; taille NS = 80 nm commerciale (ref 742023 Sigma Aldrich). M (n = 11, m = 4), L-PEG poids moléculaire = 6000, F = CH3, PEG-COOH (r = 11, p = 6).

**Figure 11** : Spectre d'absorbance de NS pré-fonctionnalisées avec des molécules M et fonctionnalisées ADN (W = 0 ou 1,6% ; Z = 12,5%) dispersées dans PBS ; M (n = 11, m = 4); taille NS = 20 nm.

**Figure 12 :** Spectre d'absorbance de NS pré-fonctionnalisées avec des molécules M (W = 10%) et fonctionnalisées avec ADN (z = 25%) dispersée dans PBS avec différents taux de recouvrement de L-PEG (X = 0 ou 10 %), F = CH3; taille NS = 20 nm. Commerciale (ref 741965 Sigma Aldrich).

**Figure 13** : Spectre d'absorbance de NS pré-fonctionnalisées avec des molécules M (W = 10 %), et fonctionnalisées avec ADN (Z = 25%) dispersées dans PBS avec différents taux de recouvrement de L-PEG (Y = 0 ou 10 %), F = CH3 ; taille NS = 80 nm commerciale (ref 742023 Sigma Aldrich).

**Figure 14** : Spectre d'absorbance de NS pré-fonctionnalisées avec des molécules M dans PBS + 0,005% surfactant en fonction du temps d'incubation ; NS de 20 nm ; temps agitation = 5, 30, 120, 240 et 1440 min, M (n = 11, m = 4), W = 40%.

**Figure 15 :** Spectre d'absorbance de NS pré-fonctionnalisées avec des molécules M et fonctionnalisées ADN (W = 4% et Z = 50%) correspondant à 3 expériences indépendantes. M (n = 11, m = 4), NP 20 nm.

**Figure 16 :** Spectre d'absorbance du surnageant de premier lavage des NS pré-fonctionnalisées avec des molécules M et fonctionnalisées ADN (W = 4% et Z = 50%) correspondant à 3 expériences indépendantes. M (n = 11, m = 4), NS 20 nm.

**Figure 17** : Spectres absorbance de NS pré-fonctionnalisées avec des molécules M après un jour, 3 et 11 mois de préparation et stockage à 4°C. NS 20 nm, M(n = 11, m = 4) avec W = 32 %.

**Figure 18** : Spectre d'absorbance de NS pré-fonctionnalisées avec des molécules M et fonctionnalisées avec des ADN thiolés après 1 jour et 2 mois de stockage à 4°C. M (n = 11, m = 4) avec W= 0 et 16 % et Z = 12,5 %.

**Figure 19 :** Spectre d'absorbance de NP pré-fonctionnalisées avec des molécules M et fonctionnalisées avec des ADN thiolés (W = 32 % et Z = 25 %) après 1 jour et 3 mois de stockage à 4°C. Taille NS = 20 nm, M (n = 11, m = 4).

**Figure 20 :** Spectre UV-Visible de NP pré-fonctionnalisées avec des molécules M et fonctionnalisées avec des ADN thiolés (W= 30%, Z = 12,5%) avant ajout de l'extrait cellulaire (trait plein) et après 1h d'incubation dans un extrait cellulaire dilué 100x (trait pointillé). Taille NS = 20 nm, M (n = 11, m = 4).

**Figure 21** : Spectre d'absorbance de l'extrait cellulaire dilué 100 x et de la somme des 3 surnageant après centrifugation (pointillé). Taille NS = 20 nm, M (n = 11, m = 4) (W = 30%, Z = 12,5%).

**Figure 22 :** Image de gauche : image de fluorescence de Cy5 afin de localiser l'ADN complémentaire fluorescent ; Image de droite : image de contraste du gel afin de localiser les nanoparticules. Taille NS = 20 nm, M (n = 11, m = 4) (W = 0 et 16 %, Z = 12,5 %).

**EXEMPLES**

Nomenclature:

- Molécule thiolée : « molécule de liaison thiolée apte à interagir avec une biomolécule » ou « biomolécule thiolée »

- W est le taux de recouvrement de la précouche constituée de molécules M.

- X est le taux de recouvrement de L-PEG

- Y est le taux de recouvrement de PEG-F

- Z est le taux de recouvrement de l'ADN thiolé

**EXEMPLE 1 : Protocole général de pré-fonctionnalisation/fonctionnalisation des nanoparticules.**

Avant toute pré--fonctionnalisation, il est préférable d'enlever l'excès de surfactant (citrate par exemple) dans les solutions de nanoparticules (NP) (diluées dans l'eau). Pour cela, au préalable, centrifuger les solutions de NP, retirer le surnageant et re-disperser le culot de NP dans un même volume d'eau distillée. La vitesse et le temps de centrifugation sont à adapter en fonction de la taille des NP.

Exemple de la préparation de NP sphériques fonctionnalisées

A partir d'une solution de NP (taille allant de 10 à 80 nm de diamètre), on effectue les étapes suivantes :

a) ajouter des molécules M pour obtenir un taux de recouvrement W compris entre 1,5 % et 99%,
b) agiter pendant une durée comprise entre 5 minutes et 4 heures,
c) de manière optionnelle, ajouter des molécules L-PEG pour obtenir un taux de recouvrement Y compris entre 1 et 10%,
d) Agiter pendant une durée comprise entre 5 et 60 min,
e) Ajouter une molécule thiolée (soit une molécule thiolée apte à interagir avec une biomolécule, soit une biomolécule thiolée) pour obtenir un taux de recouvrement Y au moins 1% ou Z = au moins 10%),
f) Agiter pendant une durée comprise entre 5 min et 24h,
g) Si la molécule thiolée est une biomolécule, se mettre dans les conditions tampons adaptées à cette dernière,
h) Centrifuger pour enlever l'excès de molécule thiolée,
i) Redisperser dans une solution appropriée (eau distillée, solution tampon avec surfactant).

**EXEMPLE 2 : La pré-fonctionnalisation de la surface des nanoparticules d'or à l'aide de molécules M améliore la stabilité de ces nanoparticules dans une solution de PBS**

a) Cas de nanoparticules sphériques

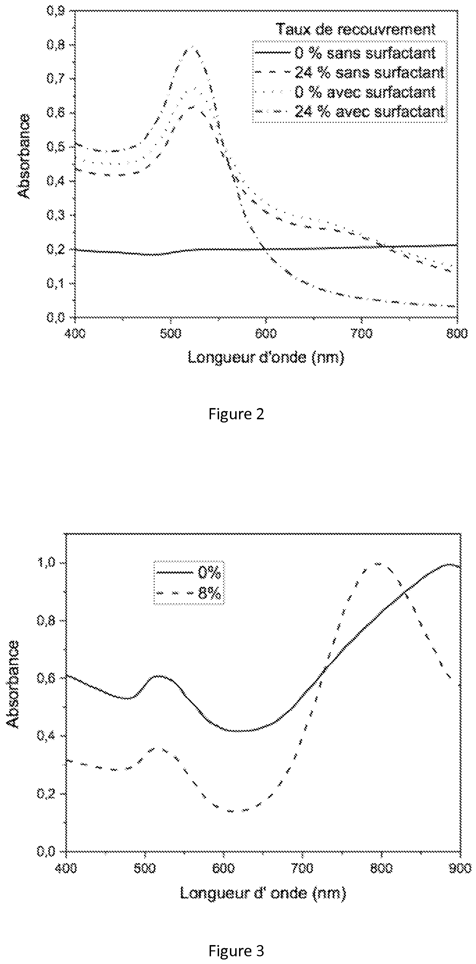

A une solution de nanoparticules, une solution de molécules M (W = 24%) est ajoutée. Le mélange est agité pendant plusieurs heures (4 heures au moins), puis est ajouté 0 et 0,005 % de surfactant, une solution tampon phosphate pH 7,4 et de NaCl. Les NPs sont alors dispersées dans PBS (Phosphate 10 mM, pH=7.4 et 0,1 M NaCl) en présence ou absence de 0,005% de surfactant.
Les données relatives à la stabilité des nanoparticules sont présentées à la Figure 2.
Nous observons que sans molécules M, les nanoparticules ne sont pas stables et s'agrègent. Au contraire, la présence de molécules M permet leur maintien en suspension. D'après les spectres d'absorbance de la Figure 2, nous observons une amélioration de la stabilité des nanoparticules dans le PBS en présence de surfactant puisque la bande plasmons s'affine.

b) Cas des nanobatônnets

A une solution de nanobâtonnets (NR), une solution de molécules M est ajoutée, puis 0,005% de surfactant. Le mélange est agité 30 minutes puis est ajouté, une solution tampon phosphate pH 7,4 et de NaCl. Les NPs sont alors dispersées dans PBS (Phosphate 10 mM, pH=7.4 et 0,1 M NaCl) en présence de 0,005% de surfactant.

Les données relatives à la stabilité des nanoparticules sont présentées à la Figure 3. Nous observons que sans molécules M, les nanoparticules ne sont pas stables et s'agrègent. Au contraire, la présence de molécules M permet leur maintien en suspension dans PBS + 0,005% surfactant.

La suite des expériences a été réalisé avec des NP sphériques.

**EXEMPLE 3** : **Les nanoparticules pré-fonctionnalisées à l'aide de molécules M peuvent être rincées et redispersées dans une solution de PBS en présence de surfactant**

A une solution de nanoparticules, une solution de molécules M est ajoutée. Le mélange est agité pendant 5 minutes ou 4 heures, centrifugé et redispersé dans une solution de PBS 1x en présence de 0,005% de surfactant.

Par « PBS 1x » on entend une solution comprenant 137 mM NaCl, 10 mM Phosphate, 2.7 mM KCl, et pH of 7,4.

Différents protocoles ont été testés :

    a) Nanoparticules sphériques de 20 nm incubées pendant 5 min dans une solution de molécules M.

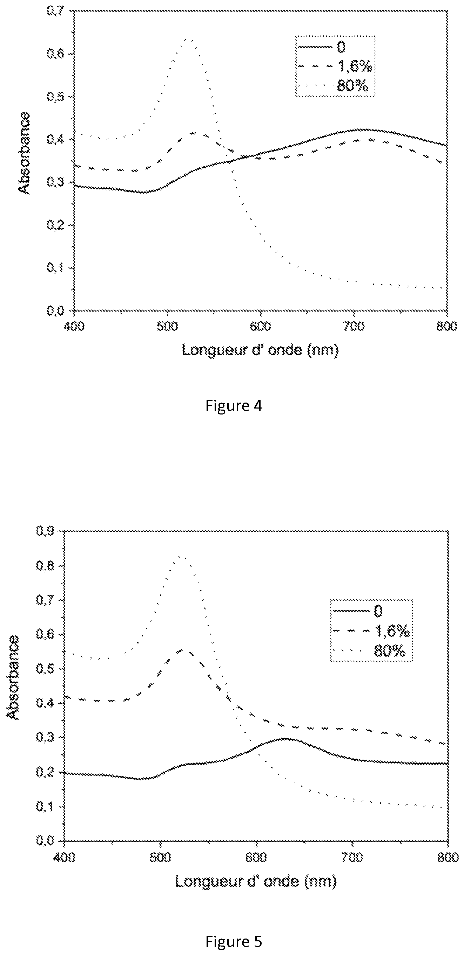

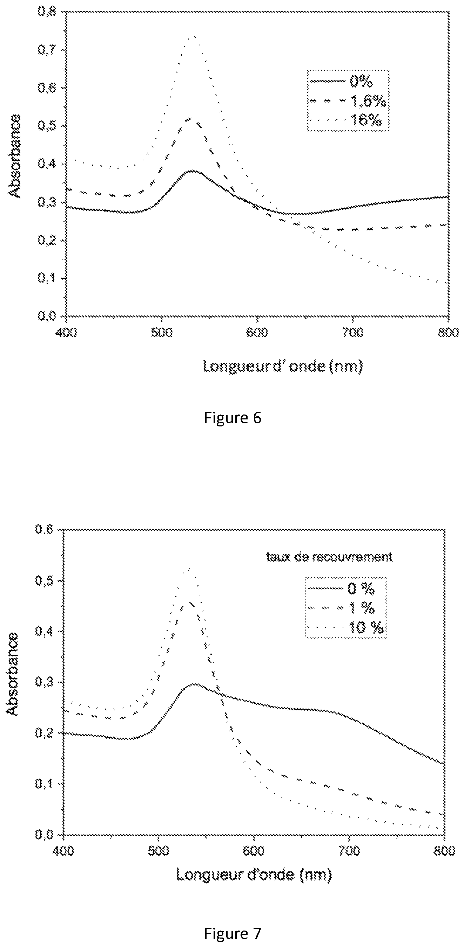

Les données relatives à la stabilité des nanoparticules sont présentées à la Figure 4.
b) Nanoparticules sphériques de 20 nm incubées pendant 4h dans une solution de molécules M.
Les données relatives à la stabilité des nanoparticules sont présentées à la Figure 5.
c) Nanoparticules sphériques de 40 nm incubées pendant 4h dans une solution de molécules M.
Les données relatives à la stabilité des nanoparticules sont présentées à la Figure 6.
Ces résultats montrent bien que la stabilité des nanoparticules augmente avec le taux de recouvrement de la solution de molécules M.

**EXEMPLE 4 : La fonctionnalisation à l'aide d'une molécule L-PEG améliore la stabilité des nanoparticules préfonctionnalisées à l'aide de molécules M dans une solution de PBS.**

a) Pré-fonctionnalisation seule

Pour la pré-fonctionnalisation seule, une solution de molécules M pour W = 1,6 à 80% a été ajoutée à une solution de nanoparticules. Le mélange a été agité pendant plusieurs minutes ou heures (5 min à 24 h), centrifugé et redispersé dans une solution appropriée.

b) Pré-fonctionnalisation suivie d'une fonctionnalisation à l'aide de molécules L-PEG

Pour la pré-fonctionnalisation suivie d'une fonctionnalisation à l'aide de molécules L-PEG, une solution de la molécule M pour obtenir un taux de recouvrement W = 5 % est ajoutée à une solution de nanoparticules. Après minimum 5 minutes d'agitation, une solution L-PEG est ajoutée pour un taux de recouvrement de 0, 1 et 10 %. Le mélange est agité encore pendant 15 min, puis centrifugé et redispersé dans une solution de PBS (1x).

Les données relatives à la stabilité des nanoparticules sont présentées à la Figure 7.

Ces résultats montrent que l'ajout de L-PEG à partir de X = 1% permet de stabiliser les NP recouvertes d'une monocouche de molécules M dans du PBS pour un faible taux de recouvrement, ici W = 5%. En effet, la bande plasmon s'affine avec L-PEG, confirmant l'amélioration de la stabilité.

**EXEMPLE 5 : La pré-fonctionnalisation de nanoparticules à l'aide de molécules M améliore la stabilité de ces nanoparticules fonctionnalisées avec une molécule thiolée dans une solution de PBS**

a) Fonctionnalisation à l'aide de molécules carboxyles thiolées sans pré-fonctionnalisation

Une solution de PEG-COOH a été ajoutée à une solution de nanoparticules de sorte à ce que le taux de recouvrement Y soit de 1,6 %. Le mélange a été agité pendant 30 minutes, centrifugé et redispersé soit dans H2O soit dans du PBS 1x + 0,005% surfactant.

b) Pré-fonctionnalisation suivie d'une fonctionnalisation à l'aide de molécules carboxyles thiolées

Une solution de molécules M a été ajoutée à une solution de nanoparticules, de sorte à ce que le taux de recouvrement théorique initial soit de 1,6% et de 80%. Après 5 minutes d'agitation, une solution de PEG-COOH a ensuite été ajoutée pour obtenir un taux de recouvrement Y de 1,6 %. Le mélange a été agité pendant 30 minutes, centrifugé et redispersé soit dans de l'eau distillée, soit dans du PBS 1x + 0,005% surfactant.

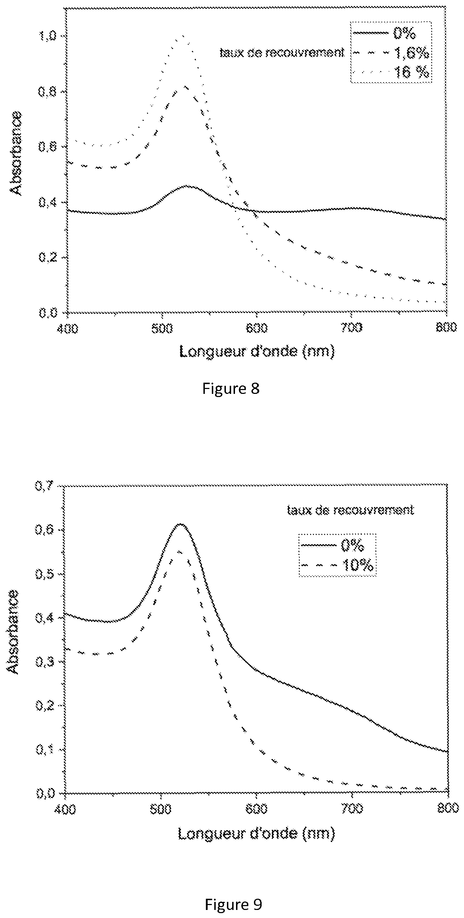

Les données relatives à la stabilité des nanoparticules sont présentées à la Figure 8.

Ces résultats montrent que les NP fonctionnalisées avec PEG-COOH seul s'agrègent dans le PBS. Le fait de pré-fonctionnaliser les NP avec une solution de molécules M permet d'améliorer la stabilité des NP en solution et ce dès un taux de recouvrement W = 1,6%. La stabilité est encore améliorée en augmentant le temps d'incubation et W, i.e. en augmentant la densité de molécules M à la surface des NPs (ici W = 16% sur la courbe reflétant la stabilité la plus élevée).

**EXEMPLE 6 : La fonctionnalisation à l'aide de molécules L-PEG améliore la stabilité des NP pré-fonctionnalisées avec des molécules M et fonctionnalisées avec une molécule thiolée dans une solution de PBS**

Une solution de molécules M a été ajoutée à une solution de NP, de sorte à obtenir un taux de recouvrement W = 5%. Une solution de L-PEG a ensuite été ajoutée de sorte à ce que le taux de recouvrement X soit 0 ou 10 %. Une solution de PEG-COOH a ensuite été ajoutée pour obtenir un taux de recouvrement Y = 5%. Le mélange a été agité, centrifugé et redispersé dans soit dans de l'eau distillée soit dans du PBS 1x + 0,005% de surfactant.

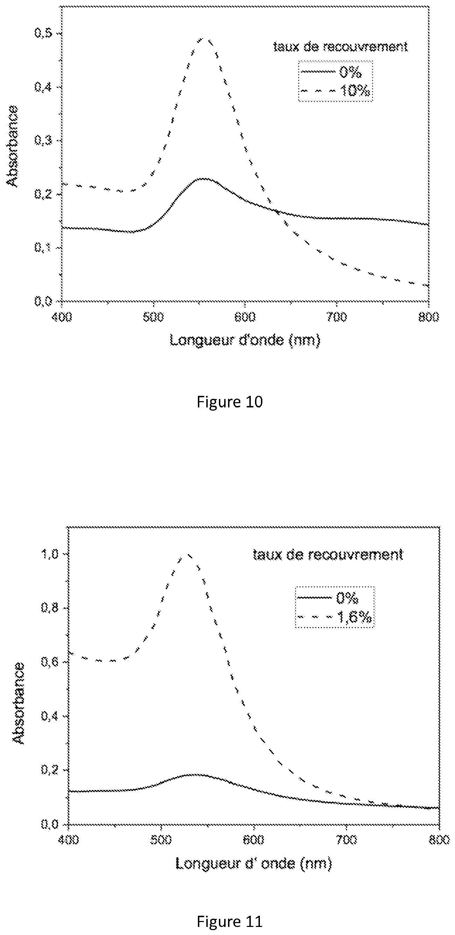

Les données relatives à la stabilité des nanoparticules sont présentées à la Figure 9 pour des NP sphériques de 20 nm et à la Figure 10 pour des NP sphériques de 80 nm.

On constate qu'en ajoutant 10 % de L-PEG, la stabilité des NP pré-fonctionnalisées et fonctionnalisées avec une molécule thiolée (ici PEG-COOH) est augmentée et ce pour de faibles taux de recouvrement de molécules M et de molécules thiolées (5% de recouvrement chacun).

**EXEMPLE 7 : La pré-fonctionnalisation à l'aide de de molécules M améliore la stabilité des NP fonctionnalisées avec une molécule thiolée dans une solution de PBS : cas d'une molécule d'ADN thiolée**

a) Fonctionnalisation à l'aide de molécules d'ADN thiolées sans pré-fonctionnalisation

Une solution d'ADN a été ajoutée à une solution de nanoparticules, pour obtenir un taux de recouvrement Z au minimum de 12,5 %. Le mélange a été agité pendant 1 heure, puis a été ajouté 0,005% de surfactant et du tampon phosphate et NaCl. Le mélange a été agité pendant 18 heures, centrifugé et redispersé dans du PBS 1x.

b) Pré-fonctionnalisation suivie d'une fonctionnalisation à l'aide de molécules d'ADN thiolées

Une solution de molécules M a été ajoutée à une solution de NP de sorte à obtenir un taux de recouvrement de 1,6 et 80%. Une solution d'ADN a ensuite été ajoutée pour obtenir un taux de recouvrement Z au minimum de 12,5%. Après 1 heure d'agitation, a été ajouté 0,005% de surfactant, du tampon phosphate et NaCl. Le mélange a été agité pendant 18 heures, centrifugé et redispersé dans du PBS 1x.

Les données relatives à la stabilité des nanoparticules sont présentées à la Figure 11.

Nous observons que les NP fonctionnalisées avec l'ADN s'agrègent dans le PBS. Toutefois, une pré-fonctionnalisation avec des molécules M améliore la stabilité à partir d'un taux de recouvrement W de 1,5%. La stabilité peut encore être améliorée en augmentant le temps d'incubation et W, i.e. en augmentant la densité de molécules M à la surface des NP (données non montrées).

**EXEMPLE 8 : La fonctionnalisation L-PEG améliore la stabilité des NP pré-fonctionnalisées avec des molécules M et fonctionnalisées à l'aide d'un ADN thiolé dans une solution de PBS**

Une solution de molécules M a été ajoutée à une solution de nanoparticules, de sorte à obtenir un taux de recouvrement W de 1,6 et de 80%. Une solution de L-PEG a ensuite été ajoutée pour obtenir un taux de recouvrement X compris entre 1 et 10 % après 30 minutes d'incubation. Le mélange a été agité pendant 15 minutes. Il a ensuite été ajouté une solution de SH-ADN pour obtenir un taux de recouvrement théorique initial de 12,5% minimum. Après ajout de la solution d'ADN, a été ajouté 0,005% de surfactant, du tampon phosphate pH 7,4 (concentration finale 10 mM) et NaCl (concentration finale 0,1 M). Le mélange a été agité pendant 18 heures, centrifugé et redispersé dans du PBS 1x.

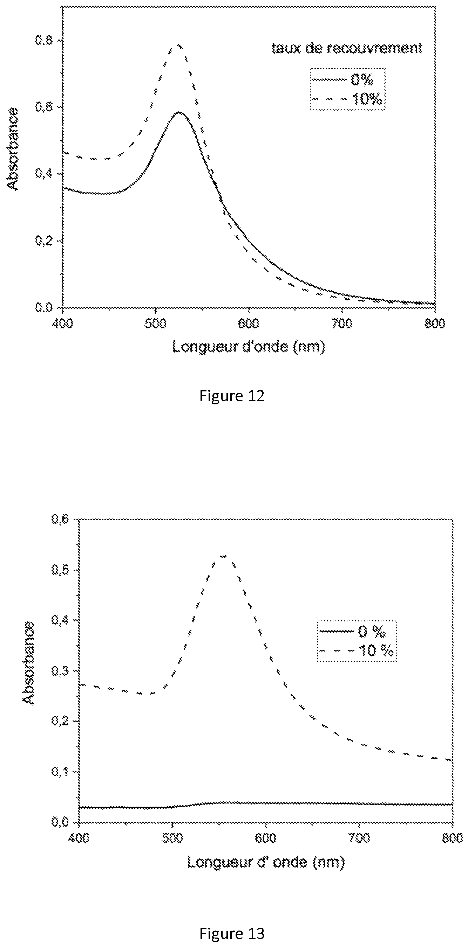

Les données relatives à la stabilité des nanoparticules sont présentées à la Figure 12 pour des NP de 20 nm et à la Figure 13 pour des NP de 80 nm.

Nous observons une amélioration de la stabilité des NP lorsque des molécules L-PEG sont ajoutées. De manière notable, la présence d'une couche de L-PEG permet de stabiliser les NP de 80 nm en solution PBS avec de faible taux de molécules M (W = 10%).

**EXEMPLE 9** : **Effet du temps d'incubation des NP dans la solution contenant la molécule M sur la stabilité**

Une solution de molécules M est ajoutée à une solution de nanoparticules. Le mélange est agité pendant plusieurs minutes ou heures (de 5 min à 24 h), puis 0,005 % de surfactant, une solution tampon phosphate pH 7,4 et de NaCl sont ajoutés. Les NPs sont alors dispersées dans du PBS (Phosphate 10 mM, pH=7.4 et 0,1 M NaCl) en présence de 0,005% de surfactant.

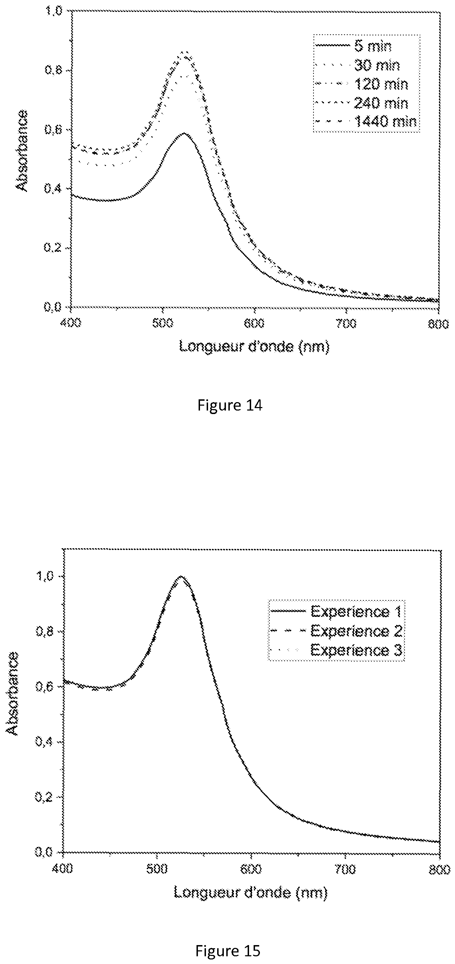

Les données relatives à la stabilité des nanoparticules sont présentées à la Figure 14.

Nous observons que le taux de recouvrement augmente en fonction du temps et atteint un maximum entre 120 et 240 min d'incubation. La poursuite de l'incubation au-delà de cette durée n'a plus s'effet sur W.

**EXEMPLE 10 : Reproductibilité de la méthode de stabilisation des nanoparticules**

Pour confirmer la reproductibilité des protocoles, ceux-ci ont été répétés trois fois à différents jours. La stabilité des échantillons a été caractérisée par spectroscopie UV-visible. Concernant le greffage de l'ADN thiolé, le 1er surnageant lors de l'étape de lavage pour enlever l'ADN en excès a été analysé par spectroscopie UV-visible.

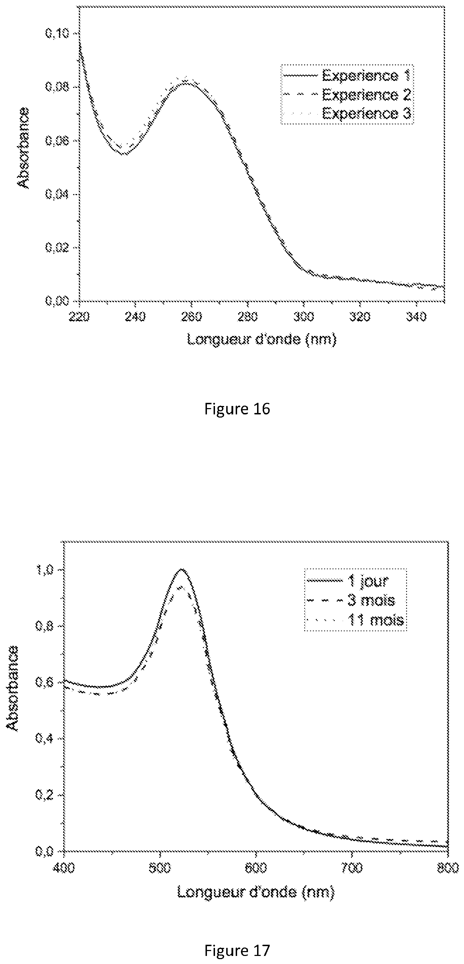

Les résultats sont présentés à la Figure 15 en ce qui concerne la stabilité de solutions de NP fonctionnalisées et à la Figure 16 en ce qui concerne le surnageant obtenu lors de la première étape de lavage de l'ADN non adsorbé.

Nous observons que les spectres UV-visibles présentent des bandes de plasmon superposables et fines, suggérant que les nanoparticules NP pré-fonctionnalisées avec des molécules M et fonctionnalisées avec de l'ADN sont stables, de même que l'intensité d'absorbance identique des surnageants suggèrent que le même nombre d'ADN a été attaché aux NPs. En conclusion, ces résultats confirment la reproductibilité dans la préparation des échantillons.

**EXEMPLE 11 : Les NP pré-fonctionnalisées avec des molécules M sont stables pendant plusieurs mois dans l'eau**

Des NP de 20 nm ont été préfonctionnalisées avec des molécules M tel que décrit précédemment puis stockées à 4° et à l'abri de la lumière pendant plusieurs mois. L'étude de stabilité a été réalisée par spectroscopie UV-visible. Typiquement, 1 mL de solution a été prélevé et analysé avec un spectromètre UVIKON. Les spectres obtenus à différents temps ont été comparés.

Les données relatives à la stabilité des nanoparticules sont présentées à la Figure 17.

Nous observons une bonne corrélation des spectres d'absorbance des NP préfonctionnalisées avec avec des molécules M après un 1 jour et 11 mois de stockage. Les trois spectres montrent des bandes de plasmon fines sans aucun déplacement de la bande plasmon, confirmant qu'après 11 mois de stockage à 4°C, les NP pré-fonctionnalisées sont toujours stables dans l'eau.

**EXEMPLE 12** : **Les NP pré-fonctionnalisées avec des molécules M et fonctionnalisées avec un ADN sont stables pendant plusieurs mois dans une solution de PBS**

Des NP de 20 nm ont été préfonctionnalisées avec des molécules M et fonctionnalisées avec un ADN tel que décrit précédemment puis stockées à 4° et à l'abri de la lumière pendant plusieurs mois. La stabilité est évaluée par spectroscopie UV-visible. Typiquement, 1 mL de solution est prélevé et analysé avec un spectromètre UVIKON. Les spectres obtenus à différents temps de stockage sont comparés.

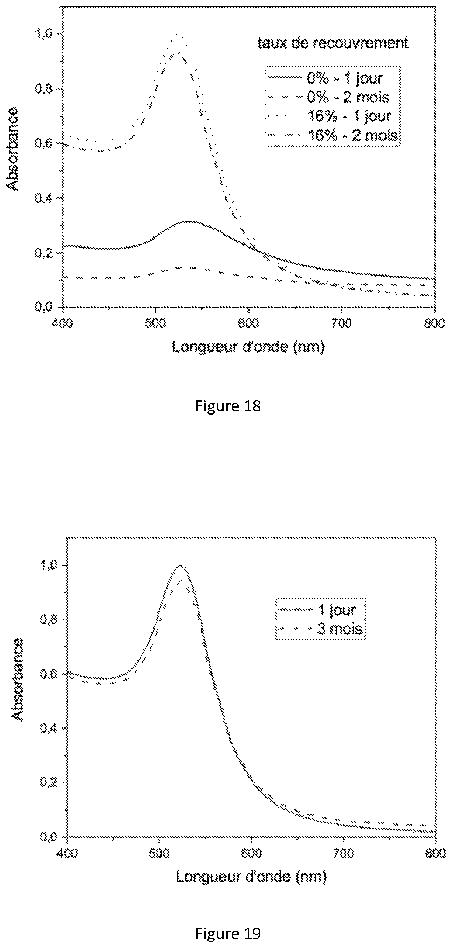

Les données relatives à la stabilité des nanoparticules sont présentées aux Figures 18 et 19.

Nous observons tout d'abord que la présence de molécules M permet de stabiliser les NP après leur préparation et pendant leur stockage. Ces résultats montrent une bonne corrélation des spectres des NP préfonctionnalisées et fonctionnalisées avec un ADN en comparant ceux obtenus après 1 jour et 2-3 mois de stockage. Les 2 spectres montrent deux bandes de plasmon fines sans aucun déplacement de la bande plasmon, confirmant qu'après 2 - 3 mois de stockage à 4°C, les NP sont toujours stables dans le PBS.

**EXEMPLE 13 : Les NP pré-fonctionnalisées et fonctionnalisées avec un ADN sont stables en milieux complexes**

10 $\mu$l d'extrait cellulaire ont été ajoutés à 1 mL de solution de NP pré-fonctionnalisées avec des molécules M et fonctionnalisées avec un ADN thiolé à 1nM (la dilution du milieu complexe est de 100 fois). Le mélange a été agité pendant 1h puis centrifugé et redispersé dans du PBS 1x. La stabilité des NP et l'intensité d'absorbance du milieu cellulaire dans les surnageants sont caractérisées par spectroscopie UV-visible. D'après la loi de Beer-lambert, l'intensité d'absorbance est liée à la concentration en solution. Si l'intensité d'absorbance de l'extrait cellulaire est similaire à l'intensité d'absorbance des surnageants, cela indique que les biomolécules présentes dans l'extrait cellulaire ne se sont pas adsorbées de façon non-spécifique aux NP.

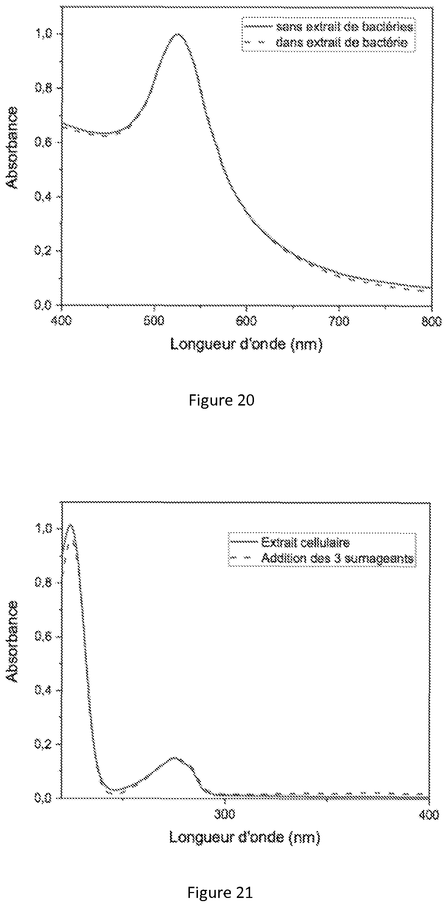

Les données relatives à la stabilité des nanoparticules sont présentées aux Figures 20 et 21.

Les spectres d'absorbance de l'échantillon avant l'ajout de l'extrait cellulaire et après 1h d'incubation ont été comparés. On remarque qu'aucune agrégation n'a lieu en milieu complexe, suggérant que les particules sont bien protégées. Afin de déterminer s'il y de l'adsorption non spécifique, nous avons lavé nos particules par centrifugation afin de retirer l'extrait cellulaire. Nous avons superposé le spectre d'extrait cellulaire initial avec la somme des 3 surnageants après les incubations d'une heure. Nous pouvons observer que les spectres se superposent, ce qui suggère qu'il y a peu ou pas d'adsorption spécifique de protéines issu de l'extrait cellulaire à la surface des NP fonctionnalisées.

**EXEMPLE 14 : La pré-fonctionnalisation des NP avec des molécules M améliore l'hybridation en milieux complexes**

1 µl d'ADN complémentaire fluorescent (Cy5) à 100 nM a été ajouté à une solution de 200 µl de NP à 1,5 nM fonctionnalisées à l'aide d'un ADN avec ou sans pré-fonctionnalisation avec des molécules M. La concentration totale en ADN complémentaire était alors de 500 pM. L'hybridation s'est déroulée pendant 2h d'incubation à 37°C dans trois milieux différents : PBS 1.x, extrait cellulaire et sérum humain. Les milieux complexes sont dilués 100 fois. Afin de différencier les ADN fluorescents, des NP fonctionnalisées avec de l'ADN et des NP préfonctionnalisées avant fonctionnalisation avec de l'ADN, les échantillons ont été déposés dans un gel d'agarose pour électrophorèse. L'électrophorèse permet de séparer et différencier les nanoparticules hybridées et les ADN complémentaires libres en solution.

Nous avons étudié l'hybridation d'un brin complémentaire fluorescent avec deux types de nanoparticules, soit NP fonctionnalisées avec des molécules d'ADN seulement, soit NP pré-fonctionalisées avec une monocouche de molécules M puis fonctionalisées avec des molécules d'ADN ,dans trois milieux différents : PBS, extrait cellulaire dilué 100x et sérum humain dilué 100x. Les nanoparticules utilisées avaient été synthétisées 2 mois avant leur utilisation et stockées à 4 °C.

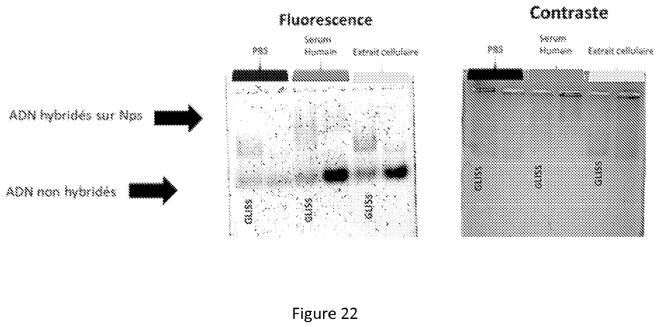

Les résultats de cette expérience sont présentés à la Figure 22.

Nous observons que seules les nanoparticules sont visibles puisque le fluorophore seul n'est pas excité. La distance de migration des nanoparticules NP fonctionnalisées avec de l'ADN et celle des NP pré-fonctionnalisées avant fonctionnalisation avec de l'ADN ne semble pas être affectée dans le PBS et l'extrait cellulaire. Par contre dans le sérum humain, la distance de migration varie très faiblement pour les NP pré-fonctionnalisées avant fonctionnalisation, mais varie de manière plus significative pour NP fonctionnalisées avec de l'ADN (sans molécules M), suggérant la possibilité d'adsorption non spécifique sur ces nanoparticules (visible par un ralentissement de la migration dû à une augmentation de la taille des NP). Sur la partie gauche de la Figure, seule l'émission de la fluorescence est observée (bandes noires dans le gel), indiquant la position de l'ADN complémentaire dans le gel. En comparant l'intensité des bandes et la position des bandes sur la Figure 22, nous pouvons corréler la position des ADNs à la position des NPs dans le gel et par conséquent l'efficacité de l'hybridation. Alors que dans le PBS, l'efficacité d'hybridation entre les nanoparticules préfonctionnalisées et celles quie n'ont pas été pré-fonctionnalisées ne semblent pas être affectée, nous observons une nette différence dans des milieux complexes. En effet, il semble que pour les échantillons sans molécules M (i.e. ADN seul) l'hybridation soit affectée puisque la fluorescence correspondant à l'ADN libre est nettement plus intense (moins d'ADN complémentaire est venu s'hybrider à l'ADN attaché aux NPs). Ces résultats suggèrent que l'efficacité d'hybridation pour l'échantillon pré-fonctionalisé est plus importante dans des milieux complexes. Nous pouvons faire l'hypothèse qu'en milieu complexe, l'adsorption non spécifique de protéines sur les NP fonctionalisées avec de l'ADN (mais non pré-fonctionalisées) empêche l'hybridation entre l'ADN complémentaire et l'ADN attaché aux NPs.

**EXEMPLE 15 : Evaluation de la détection d'ADN complémentaire cible dans différents milieux**

La détection d'un simple brin d'ADN cible (c-ADN) a été permise en suivant l'efficacité d'hybridation entre cet ADN complémentaire cible fluorescent (Cy5) et l'ADN immobilisé sur les nanoparticules pré-fonctionnalisées avec la molécule M. Une fois les ADN hybridés, deux électrophorèses sur gel ont été réalisées avant centrifugation et après centrifugation des NPs. La centrifugation permet de concentrer les NP afin d'obtenir une meilleure visualisation de l'ADN complémentaire fluorescent à la surface des NPs.

Protocole d'hybridation suivi : 50 pM à 1 nM d'ADN complémentaires fluorescents sont ajoutés dans une suspension de NPs à 2 nM (taille NPs = 20 nm, M (n = 11 et m = 4), W=16% et Z = 12,5 %). Le mélange est incubé 2h à 37 ° C soit dans le PBS, soit dans sérum humain commerciale (dilution 100) (Thermofisher, Normal Human Sérum, ref. 3.1.876).

Une migration sur gel des NP a été réalisé (données non montrées). La partie « Image de nanoparticules » montre la localisation des NP bioconjuguées et la partie « image de fluorescence » montre la localisation de l'ADN complémentaire fluorescent. Le contrôle correspond à un ADN complémentaire fluorescent libre. Différentes concentrations ont été chargées de 50 pM à 1 nM.

Ces résultats confirment que l'hybridation s'est produite puisque la fluorescence est corrélée à la position des nanoparticules. Nous observons de la fluorescence pour la plage de concentration en ADN cible allant jusqu'à 50 pM, dans du PBS ou du sérum humain. La présence de milieux complexes n'empêche pas l'hybridation, même à faible concentration en ADN cible.

Conclusion : L'hybridation sur NP de sondes ADN permet de détecter l'ADN complémentaire de 50 pM à 1 nM dans du PBS ou du sérum humain

**Revendications**

1. Nanoparticule présentant une surface métallique pré-fonctionnalisée à l'aide d'une monocouche auto-assemblée protectrice formée par une matrice de molécules M de formule (1) : $HS(CH_2)_n (OCH_2CH_2)_m OH$ dans laquelle :

   $n$ représente le nombre de $CH_2$ avec $3 \leq n \leq 11$
   $m$ représente le nombre de éthylène glycol avec $1 \leq m \leq 12$,

   ladite molécule portant une fonction thiol à l'une des extrémités, l'autre extrémité étant inerte, **caractérisé en ce que** le taux de recouvrement de ladite monocouche W est compris entre 1,5% et 99%.

2. Nanoparticule selon la revendication 1 **caractérisée en ce que** ladite nanoparticule est en outre fonctionnalisée à l'aide d'au moins une molécule L-PEG de formule (7) :

   $$SH\text{-}CH_2CH_2(CH_2CH_2O)_q O\text{-}F$$

   dans laquelle :

   $q$ représente le nombre d'éthylène glycol avec $20 \leq q \leq 500$;
   $F$ représente un groupement fonctionnel tel que $CH_3$, OH, COOH ou $NH_2$,

   le taux de recouvrement X de ladite molécule L-PEG étant d'au moins 1%.

3. Nanoparticule selon l'une des revendications 1 ou 2, laquelle est en outre fonctionnalisée à l'aide d'au moins une molécule thiolée, ladite molécule thiolée étant :

   - soit une molécule de liaison PEG-F de formule (8) : $HS(CH_2)_r (OCH_2CH_2)_p O\text{-}F$ dans laquelle :

      $r$ représente le nombre de $CH_2$ et est un nombre entier supérieur ou égal à 3.
      $p$ représente le nombre de éthylène glycol avec $2 \leq p \leq 12$, et
      $F$ représente un groupement fonctionnel tel que $CH_3$, COOH ou $NH_2$,

   ladite molécule de liaison portant une fonction thiol à l'une des extrémités et portant à son autre extrémité un groupement actif ou réactif capable d'interagir avec une biomolécule ;
   - soit une biomolécule comportant une fonction thiol.

4. Nanoparticule selon la revendication 3, **caractérisée en ce que** ladite molécule de liaison thiolée est un groupement PEG-F dans lequel F est un groupement fonctionnel COOH et dont le taux de recouvrement Y est minimum 1%.

5. Nanoparticule selon l'une des revendications 3 ou 4 **caractérisée en ce qu'**elle est en outre fonctionnalisée à l'aide d'une biomolécule liée à ladite molécule de liaison.

6. Nanoparticule selon la revendication 3, **caractérisée en ce que** ladite biomolécule thiolée est un ADN thiolé dont le taux de recouvrement Z est au minimum de 10 %.

7. Nanoparticule selon l'une des revendications précédentes, ladite nanoparticule étant une nanoparticule sphérique, un nanobâtonnet, une nanoparticule cubique un nanotriangle, coeur coquille ou un nano-oursin.

8. Procédé de préparation d'une nanoparticule présentant une surface métallique pré-fonctionnalisée à l'aide d'une monocouche auto-assemblée protectrice formée par une solution de molécules portant une fonction thiol à l'une des extrémités, l'autre extrémité étant inerte, comprenant les étapes suivantes :

- Ajouter des molécules M de formule (1) telle que définie à la revendication 1 pour obtenir un taux de recouvrement W compris entre 1,5 % et 99%,
- Incuber pendant une durée d'au moins 5 minutes

9. Procédé selon la revendication 8 comprenant en outre une étape d'ajout d'une solution de molécule L-PEG de formule (7) tel que définie à la revendication 2 pour obtenir un taux de recouvrement X compris entre 1% et 10%.

10. Procédé selon l'une des revendications 8 ou 9 comprenant en outre une étape d'ajout d'une molécule thiolée choisie parmi une molécule de liaison PEG-F de formule (8) tel que définie à la revendication 3, apte à interagir avec une biomolécule, ou une biomolécule thiolée.

11. Méthode de détection d'une molécule d'intérêt dans une solution, en particulier un milieu complexe, comprenant les étapes de :

- mise en contact d'une solution avec au moins une nanoparticule fonctionnalisée telle que définie à l'une des revendications 2 à 7,
- détection parSPR, test bandelette ou toute autre méthode appropriée, d'un signal spécifique lorsqu'une biomolécule interagit avec l'un des composants de la solution.

12. Méthode de déplétion de molécules d'intérêt présentes dans une solution, en particulier un milieu complexe comprenant les étapes de :

a) mise en contact d'une solution contenant la molécule d'intérêt avec au moins une nanoparticule fonctionnalisée telle que définie à l'une des revendications 2 à 7.
b) incubation de ladite solution en présence desdites nanoparticules
c) récupération des nanoparticules
d) optionnellement, répéter les étapes a) à c) jusqu'à épuisement de la solution en molécule d'intérêt.

13. Nanoparticule selon l'une des revendications 2 à 7 pour son utilisation dans le traitement du cancer, notamment en radiothérapie, photothérapie et pour la délivrance de médicaments, et en imagerie médicale.

**Patentansprüche**

1. Nanopartikel, der eine metallische vorfunktionalisierte Oberfläche mit einer selbstorganisierten schützenden Monolage aufweist, die durch eine Matrix aus Molekülen M der Formel (1) gebildet wird: $HS(CH_2)n (OCH_2CH_2)m OH$ wobei:

n die Anzahl von $CH_2$ mit $3 \leq n \leq 11$ darstellt
m die Anzahl von Ethylenglykol mit $1 \leq m \leq 12$ darstellt, wobei das Molekül eine Thiol-Funktion an einem seiner Endpunkte trägt, der andere Endpunkt inert ist, **dadurch gekennzeichnet, dass** die Rückgewinnungsrate der Monolage W zwischen 1,5 % und 99 % liegt.

2. Nanopartikel nach Anspruch 1, **dadurch gekennzeichnet, dass** der Nanopartikel außerdem mit mindestens einem Molekül L-PEG der Formel (7) funktionalisiert wird: $SH\text{-}CH_2CH_2(CH_2CH_2O)_qO\text{-}F$ wobei:

q die Anzahl von Ethylenglykol mit $20 \leq q \leq 500$ darstellt;
F eine funktionale Gruppe wie $CH_3$, OH, COOH oder $NH_2$ darstellt, wobei die Rückgewinnungsrate X des Moleküls L-PEG mindestens 1 % beträgt.

3. Nanopartikel nach einem der Ansprüche 1 oder 2, wobei dieser außerdem mit mindestens einem thiolierten Molekül funktionalisiert wird, wobei das thiolierte Molekül Folgendes ist:

- entweder ein Bindungsmolekül PEG-F der Formel (8): $HS(CH_2)_r (OCH_2CH_2)_p O\text{-}F$, wobei:

r die Anzahl von $CH_2$ darstellt und eine ganze Zahl größer oder gleich 3 ist.
p die Anzahl von Ethylenglykol mit $2 \leq p \leq 12$ darstellt, und
F eine funktionale Gruppe wie $CH_3$, COOH oder $NH_2$ darstellt, wobei das Bindungsmolekül eine Thiol-Funktion an einem seiner Endpunkte trägt und an seinem anderen Endpunkt eine aktive oder reaktive Gruppe trägt, die mit einem Biomolekül interagieren kann;

- oder ein Biomolekül, das eine Thiol-Funktion enthält.

4. Nanopartikel nach Anspruch 3, **dadurch gekennzeichnet, dass** das thiolierte Bindungsmolekül eine Gruppe PEG-F ist, wobei F eine funktionale Gruppe COOH ist und dessen Rückgewinnungsrate Y minimal 1 % beträgt.

5. Nanopartikel nach einem der Ansprüche 3 oder 4,
**dadurch gekennzeichnet, dass** es außerdem mit einem Biomolekül, das sich mit dem Bindungsmolekül verbindet, funktionalisiert wird.

6. Nanopartikel nach Anspruch 3, **dadurch gekennzeichnet, dass** das thiolierte Biomolekül eine thiolierte DNA ist, deren Rückgewinnungsrate Z minimal 10 % beträgt.

7. Nanopartikel nach einem der vorstehenden Ansprüche, wobei der Nanopartikel ein sphärischer Nanopartikel, ein Nanostäbchen, ein kubischer Nanopartikel, ein Nanodreieck, Kern-Hülle-Nanopartikel oder ein Nano-Seeigel ist.

8. Verfahren zum Herstellen eines Nanopartikels, das eine metallische vorfunktionalisierte Oberfläche mit einer selbstorganisierten schützenden Monolage aufweist, die durch eine Lösung von Molekülen gebildet wird, die eine Thiol-Funktion an einem ihrer Endpunkte tragen, wobei der andere Endpunkt inert ist, umfassend die folgenden Schritte:

- Hinzufügen der Moleküle M der Formel (1), wie in Anspruch 1 definiert, um eine Rückgewinnungsrate W zwischen 1,5 % und 99 % zu erhalten,
- Inkubieren für eine Zeitdauer von mindestens 5 Minuten

9. Verfahren nach Anspruch 8, umfassend außerdem einen Schritt des Hinzufügens einer Lösung des Moleküls L-PEG der Formel (7), wie in Anspruch 2 definiert, um eine Rückgewinnungsrate X zwischen 1 % und 10 % zu erhalten.

10. Verfahren nach einem der Ansprüche 8 oder 9, umfassend außerdem einen Schritt des Hinzufügens eines thiolierten Moleküls, ausgewählt aus einem Bindungsmolekül PEG-F der Formel (8), wie in Anspruch 3 definiert, geeignet, um mit einem Biomolekül zu interagieren, oder einem thiolierten Biomolekül.

11. Methode zum Nachweisen eines Moleküls von Interesse in einer Lösung, insbesondere in einem komplexen Umfeld, umfassend die Schritte von:

- Inkontaktbringen einer Lösung mit mindestens einem funktionalisiertem Nanopartikel, wie in einem der Ansprüche 2 bis 7 definiert,
- Nachweisen durch SPR, Teststreifen oder jeder anderen geeigneten Methode, eines spezifischen Signals, wenn ein Biomolekül mit einer der Komponenten der Lösung interagiert.

12. Methode zum Vermindern der Moleküle von Interesse, die in einer Lösung vorhanden sind, insbesondere in einem komplexen Umfeld, umfassend die Schritte von:

a) Inkontaktbringen einer Lösung, die das Molekül von Interesse enthält, mit mindestens einem funktionalisierten Nanopartikel, wie in einem der Ansprüche 2 bis 7 definiert.
b) Inkubieren der Lösung in Anwesenheit der Nanopartikel
c) Rückgewinnen der Nanopartikel
d) optional Wiederholen der Schritte a) bis c) bis zum Ausschöpfen der Lösung mit dem Molekül von Interesse.

13. Nanopartikel nach einem der Ansprüche 2 bis 7 für seine Verwendung in der Behandlung von Krebs, insbesondere bei der Strahlentherapie, Phototherapie und für die Abgabe von Arzneimitteln und bei der medizinischen Bildgebung.

**Claims**

1. Nanoparticle having a pre-functionalized metal surface using a self-assembled protective monolayer formed by a matrix of M molecules of the formula (1): $HS(CH_2)n\ (OCH_2CH_2)m\ OH$
   where:

   n represents the number of $CH_2$ with $3 \leq n \leq 11$
   m represents the number of ethylene glycol with $1 \leq m \leq 12$, said molecule bearing a thiol function at one of its ends, the other end being inert,

   **characterized in that** the coverage rate of said monolayer W is between 1.5% and 99%.

2. Nanoparticle according to claim 1, **characterized in that** said nanoparticle is further functionalized using at least one L-PEG molecule of the formula (7): $SH\text{-}CH_2CH_2(CH_2CH_2O)_qO\text{-}F$
   where:

   q represents the number of ethylene glycol with $20 \leq q \leq 500$;
   F represents a functional group such that $CH_3$, OH, COOH or $NH_2$, the coverage rate X of said L-PEG molecule being at least 1%.

3. Nanoparticle according to any of claims 1 or 2, which is further functionalized using at least one thiolated molecule, said thiolated molecule being:

   - either a PEG-F binding molecule of the formula (8): $HS(CH_2)r\ (OCH_2CH_2)_P\ O\text{-}F$ where:

     r represents the number of $CH_2$ and is an integer greater than or equal to 3.
     p represents the number of ethylene glycol with $2 \leq p \leq 12$, and
     F represents a functional group such that $CH_3$, COOH or $NH_2$, said binding molecule bearing a thiol function at one of its ends and bearing at its other end an active or reactive group capable of interacting with a biomolecule;

     - or a biomolecule comprising a thiol function.

4. Nanoparticle according to claim 3, **characterized in that** said thiolated binding molecule is a PEG-F group, F being a COOH functional group and of which the coverage rate Y being at least 1%.

5. Nanoparticle according to any of claims 3 or 4, **characterized in that** it is further functionalized using a biomolecule bound to said binding molecule.

6. Nanoparticle according to claim 3, **characterized in that** said thiolated biomolecule is a thiolated DNA of which the coverage rate Z is at least 10%.

7. Nanoparticle according to any of the preceding claims, said nanoparticle being a spherical nanoparticle, a nanorod, a cubic nanoparticle, a nanotriangle, core shell nanoparticle, or a nano-urchin.

8. Method for preparing a nanoparticle having a pre-functionalized metal surface using a protective self-assembled monolayer formed by a solution of molecules bearing a thiol function at one of their ends, the other end being inert, comprising the following steps:

   - adding M molecules of the formula (1) as defined in claim 1 to obtain a coverage rate W of between 1.5% and 99%,
   - incubating for a period of at least 5 minutes.

9. Method according to claim 8, further comprising a step of adding an L-PEG molecule solution of the formula (7) as defined in claim 2 to obtain a coverage rate X of between 1% and 10%.

**10.** Method according to any of claims 8 or 9, further comprising a step of adding a thiolated molecule selected from a PEG-F binding molecule of the formula (8) as defined in claim 3, which is capable of interacting with a biomolecule or a thiolated biomolecule.

**11.** Method for detecting a molecule of interest in a solution, in particular a complex medium, comprising the steps of:

- contacting a solution with at least one functionalized nanoparticle as defined in any of claims 2 to 7,
- detecting, via SPR, strip test or any other suitable method, a specific signal when a biomolecule interacts with one of the components of the solution.

**12.** Method for depleting molecules of interest present in a solution, in particular a complex medium comprising the steps of:

a) contacting a solution containing the molecule of interest with at least one functionalized nanoparticle as defined in any of claims 2 to 7.
b) incubating said solution in the presence of said nanoparticles
c) collecting the nanoparticles
d) optionally repeating steps a) to c) until the solution with the molecule of interest is exhausted.

**13.** Nanoparticle according to any of claims 2 to 7 for its use in the treatment of cancer, in particular in radiotherapy, phototherapy and for drug delivery, and in medical imaging.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2011165077 A
- US 2019142966 A
- US 2016243254 A

**Littérature non-brevet citée dans la description**

- **HURST K.M. et al.** *Journal of Microelectromechanical Systems,* Avril 2011, vol. 20 (2
- **WANG et al.** *RSC Adv.,* 2017, vol. 7, 3676-3679
- **LI et al.** *Langmuir,* 2015